# EUROPEAN PATENT APPLICATION

(11) **EP 3 782 593 A1**
(43) Date of publication of application: **24.02.2021**
(21) Application number: 19203408.0
(22) Date of filing: 15.10.2019
(51) Int. Cl.: A61F 13/20, A61F 13/34, A61B 10/02

(54) **TAMPON ASSEMBLY**

(30) Priority: 23.08.2019 US 201962922712 P
(71) Applicant: Edmunds, Kathleen, Knoxville, TN 37919 (US)
(72) Inventor: Edmunds, Kathleen, Knoxville, TN 37919 (US)
(74) Representative: Boult Wade Tennant LLP

(57) **Abstract**

A tampon assembly (20) includes a relatively inflexible impermeable portion (22) having two opposite concave and convex side faces (28, 30) and an absorbent portion (24) positioned adjacent the concave side face of the impermeable portion includes a tension member (100) for facilitating the removal of the assembly following use. The tension member has two opposite ends (74, 76) and a major portion (78) which extends between the ends. The major portion is sandwiched between the concave side face and the absorbent portion, one of the two opposite ends of the tension member is secured to the absorbent portion, and the other of the two opposite ends of the tension member extends through an opening (90 or 91) defined within the impermeable portion and is accessible to a user. By providing the absorbent material with an abrasive surface (229), the assembly can be utilized for collecting cells for laboratory purposes.

## Description

### BACKGROUND OF THE INVENTION

This invention relates generally to feminine hygiene products and relates, more particularly, to tampon devices.

The class of tampon devices with which the present invention is concerned includes those which include, in assembly form, a liquid-impermeable portion and an absorbent portion which is held against a side face of the impermeable portion. The absorbent portion is adapted to absorb fluids which may exit the cervix of the user while the impermeable portion of the assembly is adapted to provide a satisfactory seal between the outer edges of the impermeable portion and the walls of the vaginal canal to prevent passage of fluids past the impermeable portion. An example of a tampon device, or assembly, of this class is shown and described in U.S. Pat. 9,357,982 naming the same inventor as the present invention and which is incorporated herein by reference.

Heretofore, strings have been utilized with tampon assemblies of the aforedescribed class to facilitate removal of the device from the vaginal canal. That is to say that the withdrawal of the tampon assembly from the vaginal canal is effected by pulling upon the string. When such string-including assemblies are positioned in place, the string commonly extends along the vaginal canal toward the opening thereof. However, it would be desirable to provide such a string-including assembly whose string remains positioned in an out-of-the-way condition until such time as it is needed for assembly removal purposes.

Accordingly, it is an object of the present invention to provide a new and improved tampon assembly of the aforedescribed class which possesses a string or some other tension member to facilitate the removal of the assembly from a vaginal canal.

Another object of the present invention is to provide such an assembly whose string, or a major portion of the length thereof, does not extend along the length of the vaginal canal while the assembly is positioned in place for use.

Yet another object of the present invention is to provide such an assembly whose string is positioned, or gathered, in an out-of-the-way condition until needed for purposes of removing the assembly from the canal.

It has been described in the referenced patent that an assembly of this class could be used to collect cervical cells of a patient for laboratory (e.g. pap smear) testing purposes. However, it has been found that some materials comprising the absorbent portion of a tampon assembly of this class is incapable of reliably collecting cervical cells. Accordingly, it would be desirable to provide a tampon assembly of this class which increases the likelihood that the tampon assembly will collect cervical cells for testing purposes.

Accordingly, a further object of the present invention is to provide a new and improved tampon assembly for collecting cervical cells for laboratory (e.g. pap smear) testing purposes.

A yet further object of the present invention is to provide such an assembly including an absorbent material having an enhanced capacity for collecting cervical cells for laboratory testing purposes.

A still further object of the present invention is to provide such an assembly which is uncomplicated in structure, yet effective in operation.

### SUMMARY OF THE INVENTION

This invention resides in a tampon assembly, and the tampon assembly includes a thin saucer-shaped impermeable portion having two opposite concave and convex side faces and having an outer edge which is oval in shape and markedly elongate in form. The concave side face has an interior and the impermeable portion includes a leading end which first enters a user when positioned therein and an opposite trailing end, and the impermeable portion defines an opening therein adjacent the trailing end thereof. In addition, an absorbent portion is positioned adjacent the concave side face of the impermeable portion, and there is provided a tension member facilitating the withdrawal of the assembly following use. The tension member has two opposite ends and a major portion which extends between the two opposite ends, and the major portion of the tension member is positioned within the interior of the concave side face of the impermeable portion. Moreover, one end of the two opposite ends of the tension member protrudes through the through-opening and is accessible to a user, and the other of the two opposite ends of the tension member is prevented from exiting the interior of the concave side face through the through-opening so that by pulling upon the one end of the two opposite ends of the tension member during an assembly removal process withdraws the tension member from the interior of the concave side face to a fully extended condition before the remainder of the assembly is withdrawn from the user.

### BRIEF DESCRIPTION OF THE DRAWINGS

Fig. 1 is a perspective view of an embodiment of a tampon assembly within which features of the present invention are embodied and shown exploded.
Fig. 2 is a longitudinal cross-sectional view of the Fig. 1 assembly, shown exploded.
Fig. 3 is a longitudinal cross-sectional view of the Fig. 1 assembly, shown assembled.
Fig. 4 is a plan view of the impermeable portion (with the absorbent portion removed therefrom) and the tension member of the Fig. 1 assembly as seen generally from above in Fig. 1 and depicting the relationship between the tension member and the impermeable portion before the tension member is pulled upon by a user.
Fig. 5 is a plan view similar to that of Fig. 4 depicting the relationship between the tension member and the impermeable portion of the Fig. 1 assembly when the tension member is pulled to a fully extended condition.
Fig. 6 is a cross-sectional view illustrating schematically the manner in which the Fig. 1 assembly is positioned for use.
Fig. 7 is a perspective view of an alternative absorbent portion capable of being used within the assembly of the present invention.

### DETAILED DESCRIPTION OF AN ILLUSTRATIVE EMBODIMENT

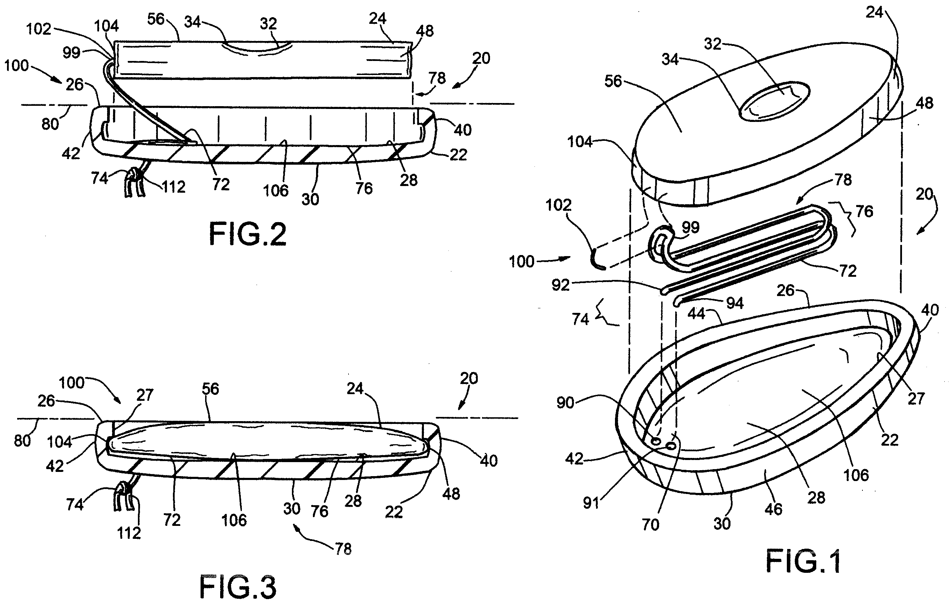

Turning now to the drawings in greater detail and considering first Fig. 1, there is illustrated an embodiment, generally indicated 20, of a tampon assembly within which features of the present invention are embodied. The assembly 20 includes a liquid-impermeable portion 22 and an absorbent pad portion 24 which is releasably secured to the impermeable portion 22. As will be apparent herein, the absorbent portion 24 is adapted to absorb fluids which may exit the cervix of the user while the impermeable portion 22 of the tampon assembly 20 is adapted to provide a satisfactory seal between the outer edges of the impermeable portion 22 and the walls of the vaginal canal to prevent passage of fluids past the impermeable portion 22. In addition, the impermeable portion 22 is shaped and sized to facilitate the insertion of the assembly 20 into place within the vaginal canal for use and the removal of the assembly 20 from the vaginal canal following use without the need that the assembly 20, or more specifically, the impermeable portion 22 be compressed during insertion or removal.

Although the assembly 20 is primarily described herein as being used for preventing the discharge of fluids from the vaginal canal, the assembly 20 can also be used in applications in which it is desired to strengthen the vaginal walls (from the inside thereof) to prevent deformation or displacement thereof. For example, there exists physical conditions, such as bladder prolapse, cervical prolapse or rectum prolapse, characterized by the displacement of various organs from their normal positions and which, in some instances, bear upon the vaginal walls. To counter the deleterious effects of such physical conditions, the assembly 20 can be positioned in its desired position along the vaginal canal to strengthen the walls of the vaginal canal and thereby resist displacement of the organs. Accordingly, the assembly 20 can be used as a substitute for a pessary which might otherwise be used as an aid in this regard.

Furthermore, the assembly 20 can also be used (in a form described herein) to collect cells for laboratory purposes. That is to say that a form of the assembly 20 can be used to collect cervical cells upon the surface of the absorbent portion 22, and such collected cells can be used for laboratory (e.g. pap smear) testing purposes. Accordingly, the principles of the present invention can be variously applied.

With reference to Figs. 1-5, the impermeable portion 22 is saucer-shaped in form having a relatively shallow, concave side face 28 and an opposite convex side face 30. In addition, the impermeable portion 22 is relatively thin as measured between the side faces 28, 30. Moreover, the impermeable portion 22 is substantially inflexible in nature so as to provide a degree of stiffness or rigidity to the assembly 20 when positioned in place within the vaginal canal for use. Preferably, the impermeable portion 22 is molded with integral ribs (or regions of increased thickness) which extend longitudinally along the concave side face 28 of the impermeable portion 22. As will be apparent herein, the stiffness or rigidity of the impermeable portion 22 is advantageous in that it helps to rigidify the assembly 20 and strengthen the walls of the user's vaginal canal when the assembly 20 is positioned therein.

To provide the impermeable portion 22 with a desired degree of inflexibility, the impermeable portion 22 can be constructed of any of a number of elastomeric materials, such as a relatively hard plastic (which can include silicon), or other classes of materials, such as a coated paper. However, it may be preferable to avoid latex as a choice of material for the impermeable portion 22 due to the allergic reaction that some individuals have to that material.

It is a feature of the assembly 20 that its impermeable portion 22 has a markedly oval-shaped outer edge 26 (defining an inwardly-directed lip 27) to provide a better-fitting relationship between the outer edge 26 and the walls of the vaginal canal than can be had with circular-shaped portions of diaphragm-class of tampons of the prior art. In this connection, applicant, who is an obgyn physician, has discovered that in most females whom she has examined, the anterior-posterior dimension of the pelvis is greater than the transverse diameter of the pelvis. In other words and in order for the impermeable portion 22 to suitably fit within the vaginal canal and provide a desirable and comfortable sealing relationship with the walls of the canal, applicant has discovered that the front-to-back dimension of the impermeable portion 22 should be larger than the side-to-side dimension of the impermeable portion 22.

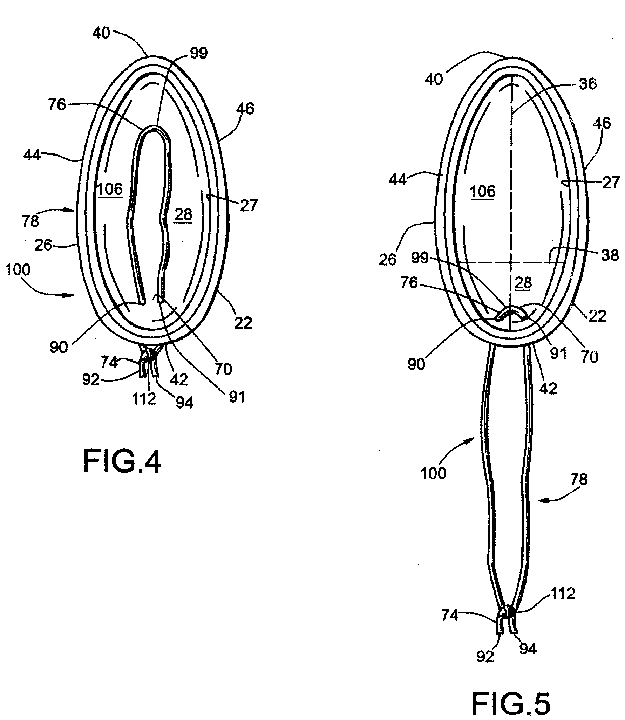

More specifically, the outer edge 26 of the impermeable portion 22 of the depicted assembly 20 (as best viewed in Fig. 5) is shaped to resemble an oval of markedly elongate form so that its maximum dimension (as measured along its major, or longitudinal, axis 36, indicated in phantom and extending through the center of the oval shape of the outer edge 26) is at least about 1.5 times the size of the minimum dimension (as measured along its minor axis 38, also indicated in phantom and extending through the center of the oval shape of the outer edge 26). Preferably, the oval-shaped outer edge 26 has a maximum dimension which is at least about 2.0 times the size of its minimum dimension. It has been found that the depicted assembly 20, with its oval-shaped impermeable portion 22, provides a better edge-to-vaginal canal sealing relationship than is capable of being formed with any impermeable portion of circular form and is advantageous in this respect.

While an oval, by definition, is symmetrical about at least one of its major or minor axes, the oval shape of the impermeable portion 22 could be symmetrical about each of its major and minor axes 36, 38. However, the shape of the depicted impermeable portion 22 somewhat resembles that of a teardrop having one (i.e. a leading) end 40 which is smaller, or more pointed in form, than the opposite (i.e. trailing) end 42 thereof.

Moreover and as used herein, the phrase "oval shape of markedly elongate form" is intended to connote that the outer edge 26 of the impermeable portion 22 has an appearance which is distinctively elongate, and such a distinctively elongate appearance is described herein in structural terms by way of the stated requirement that the maximum diameter of the impermeable portion 22 as measured along its major axis 36 is at least 1.5 times the size of the minimum dimension of the impermeable portion 22 as measured along its minor axis 38.

The assembly 20, and more specifically, the impermeable portion 22, is intended to be inserted endwise, or lengthwise, into place through the vaginal canal as the impermeable portion 22 is directed and moved lengthwise (i.e. in a direction parallel to the major axis 36) through the canal. Accordingly and for present purposes, the leading end of the impermeable portion 22 (i.e. the end of the impermeable portion 22 which is inserted first through the vaginal canal into place therein) is indicted 40 in Figs. 1-4, the opposite, or trailing, end of the impermeable portion 22 (i.e. the end of the impermeable portion 22 which follows the leading end 40 into place) is indicated 42 in Figs. 1-4, and the opposite sides of the impermeable portion 22 which extend between the leading and trailing ends 40 and 42 are indicated 44 and 46 in Figs. 1-4.

With reference again to Figs. 1-3, the absorbent portion 24 is comprised of a substantially platen arrangement of soft absorbent material, such as a layup of cotton, rayon or a cotton/rayon blend of fibers, and is positionable against the concave side face 28 of the impermeable portion 22 for securement thereto. As best shown in Fig. 1, the absorbent portion 24 has an outer edge 48 which is shaped similar (i.e. oval-shaped) to that of the outer edge 26 and is centrally-disposed against the side face 28 of the impermeable portion 22 (and thus within the interior, indicated 106, of the concave side face 28) so that the outer edge 48 is captured beneath the inwardly-directed lip 27 of the impermeable portion 22, as best shown in Fig. 3 so that the absorbent portion 24 is thereby secured to the impermeable portion 22. Accordingly, the outer edge 48 of the absorbent portion 24 is slightly smaller in size than the outer edge 26 so that when positioned against the concave side face 28, no part of the absorbent portion 24 extends outboard of the outer edge 26. With the absorbent portion 24 thus positioned against the side face 28, the total thickness of the assembly 20, as measured at its thickest point, is preferably no more than about 2.0 cm.

If desired, there can be provided in the upwardly-facing surface, indicated 56, of the absorbent portion 22 a pre-formed concave indentation 32 which is centrally disposed therein. Within the depicted assembly 20, the concave indentation 32 has an outer, oval-shaped edge 34 which is concentrically-arranged within the outer edge 26 of the impermeable portion 22 and possesses a dimension along each of its major and minor axes which is about one-third the size of the corresponding major or minor axis 36 or 38 of the outer edge 32. Meanwhile, the depth of the concave indentation 34 (as measured from the plane of the surface 56) is about one centimeter, but the indentation 34 can possess an alternative depth.

It is a feature of the assembly 20 that it is provided with a tension member, generally indicated 100, which is intended to aid the removal of the assembly 20 from the user following use yet remains, for the most part, sandwiched between the concave side face 28 of the impermeable portion 22 and the absorbent portion 24 until pulled upon during an assembly removal process. In this connection and with reference to Figs. 4 and 5, the impermeable portion 22 is provided with a pair of through-openings 90, 91 which extend between the concave and convex side faces 28, 30 thereof and which are disposed in a side-by-side-relationship adjacent the trailing end 42 of the impermeable portion 22. In addition, the through-openings 90, 91 are spaced relatively close to one another and are separated from one another by a region, indicated 70 in Fig. 1, of the impermeable portion 22.

With reference to Figs. 1, 4 and 5, the tension member 100 has two opposite ends 74, 76 and a major portion 78 which extends between the ends 74, 76. One of the ends 74 extends out of the impermeable portion 22 through the through-openings 90, 91 and is accessible to a user, and the other of the ends 76 is prevented from exiting the impermeable portion 22 when the one end 74 is pulled upon by user. Meanwhile, the major portion 78 of the tension member 100 is positioned within the interior 106 of the concave side face 28 of the impermeable portion 22. As will be apparent herein, by pulling upon the one end 74 of the two opposite ends 74, 76 of the tension member 100 during an assembly removal process withdraws the tension member 100 from the interior of the concave side face to a fully extended condition before the remainder of the assembly 20 is withdrawn from the user.

The tension member 100 of the depicted assembly 20 is in the form of a cord, or string, 72 which is comprised of a material (e.g. cotton) which is flexible in nature and has two opposite end portions 92, 94 which are folded back upon themselves so that one end 74 of the tension member 100 is provided by the two end portions 92, 94 positioned alongside one another (and tied in a knot 112) and so that the other end 76 of the tension member 100 is provided by a looped mid-portion, indicated 99, of the cord 72.

During assembly of the assembly 20, the looped mid-portion 99 of the cord 100 is preferably secured, as with a stitch 102 of thread, to an end, indicated 104, of the absorbent portion 24 intended to be positioned adjacent (i.e. within) the trailing end 42 of the absorbent portion 24 when the absorbent portion 24, and the two ends 92, 94 of the cord 72 are directed endwise for a short distance (e.g. less than about 0.5 inches) through the through-openings 90, 91 from the interior 106 of the concave side face 28 and tied in a knot 112 external, or outside, of the interior 106 of the concave side face 28 of the impermeable portion 22. As will apparent herein, the knot 112 provides a user with a pull tab capable of being grasped (i.e. by a user) for purposes of removing the assembly 20 following use, and the looped mid-portion 99 - when the tension member 100 is pulled from the gathered condition, as best shown in Fig. 4, to a fully extended condition, as best shown in Fig. 5, acts upon the region 70 of the impermeable portion 22 disposed between the through openings 90, 91 so that the impermeable portion 22 is drawn from the vaginal canal by way of the tension member 100. It follows that the end 76 of the tension member 100 is thereby prevented from exiting the interior 106 of the concave side face 28.

It is another feature of the assembly 20 that the major portion 78 of the length of the tension member 100 remains positioned between the concave side face 28 of the impermeable portion 22 and (the underside of) the absorbent portion 24 until such time as it is desired to remove the assembly 20 following use. Preferably, the major portion 78 of the tension member 100 remains positioned in a gathered condition adjacent the concave side face 28. Such a gathered condition could take the form of, for example, a looped, folded, wound or linear arrangement, as depicted in Fig. 4, to reduce the likelihood that the major portion 78 of the tension member 100 will become knotted when pulled (by way of the knot 112) to the fully-extended condition of Fig. 5 through the through-openings 90, 91, but the gathered condition of the major portion 78 could take an alternative form.

When assembling the assembly 20, the major portion 78 of the tension member 100 is positioned in a gathered condition within the interior 106 of the concave side face 28 prior to the placement of the absorbent portion 24 within the interior 106 of the concave side face 28 and across, or atop, the major portion 78.

It follows that with the absorbent portion 24 secured with the impermeable portion 22 (as the outer edges 48 of the absorbent portion 24 are held beneath the lip 27 of the impermeable portion 22), the major portion 78 of the tension member 100 is sandwiched between the concave side face 28 and the absorbent portion 24.

Because the trailing end 42 of the impermeable portion 22 has been described above as being the end of the impermeable portion 22 which follows the leading end 40 of the impermeable portion 22 into the vaginal canal during installation, the trailing edge 42 is the first end of the impermeable portion 22 which exits the vaginal canal when withdrawn from the user. This being the case, the knot 112 provides a visual indication to the user as to which end of the impermeable portion 22 corresponds with the trailing end 42 thereof.

If desired, the entirety of the assembly 20 (i.e. its impermeable portion 22 and absorbent portion 24) can be coated with a thin coating 68 (Fig. 1) of lubricant, such as petroleum jelly, to facilitate the insertion and removal of the assembly 20.

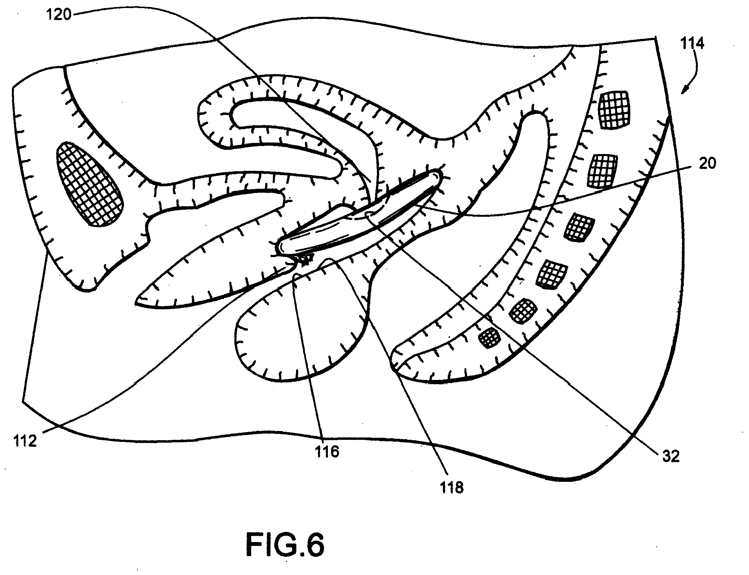

With reference to Fig. 6, there is schematically illustrated a cross-sectional portion, indicated 114, of the female anatomy within which the Fig. 1 assembly 20 can be positioned for use. More specifically, the depicted portion 114 includes a vaginal canal 116 having tubular walls 118 and a cervix 120 disposed adjacent the distal, or rearward, end of the vaginal canal 116. When fully positioned within the vaginal canal 116, the outer edge 26 of the impermeable portion 22 bears and rests against the vaginal walls 118 from about the posterior fornix (the vaginal region disposed rearwardly of the cervix 120) to the sub-pubic portion of the upper vaginal wall (i.e. a location disposed immediately behind the pubic bone) and the opening-defining end of the cervix 120 is accepted by the concave indentation 32 of the absorbent portion 24. In addition, the knot 112 of the tension member 100 which is accessible to a user for withdrawal of the assembly 20 following use does not extend far from the impermeable portion 22 when the assembly 20 is in place. This fact - and the fact that the major portion 78 of the tension member 100 remains internal of the impermeable portion 22 until the tension member end 74 is pulled upon by a user) reduces any likelihood that the tension member 100 will interfere with intercourse, and the assembly 20 is further advantageous in this respect.

As suggested earlier, the assembly 20 is inserted into place into the vaginal canal for use as the impermeable portion 22 is directed endwise (i.e. leading end 40-first) into and through the vaginal canal 116. The relatively small size of the smallest dimension (i.e. as measured across the minor axis 38) of the oval-shaped impermeable portion 22 in comparison to the size of the largest, or lengthwise, dimension (i.e. as measured across the major axis 36) of the impermeable portion 22 facilitates the lengthwise movement of the assembly 20 along the canal without requiring that the impermeable portion 22 be compressed across the smallest dimension during insertion of the assembly 20 into place or during removal of the assembly 20 following use.

With the assembly 20 disposed in position within the vaginal canal 116 for use, the contributions of the oval shape of the outer edge 26 of the impermeable portion 22 and the engagement of the outer edge 26 against the vaginal walls 118 places the outer edge 26 of the impermeable portion 22 in satisfactory sealing relationship with the vaginal walls 118. Furthermore, any discharge from the cervix 120 (whose opening-defining end effectively self-centers itself within the concave indentation 32) is permitted to pool or collect within the indentation 32 for absorption by the material of the absorbent portion 24. Therefore and during use, the absorbent portion 24 is in position to absorb fluid discharged from the cervix 120, even though the cervix 120 is not encapsulated by (the outer edges 26) of the impermeable portion.

Moreover, as the absorbent portion 24 is permitted to expand due to its absorption of fluids exiting the cervix 120, the absorbent portion 24 is prevented from expanding in every direction with respect to the vaginal canal 116, and instead, its expansion is confined to a direction which is substantially away from the concave side face 28. More specifically, the absorbent portion 24 is prevented from expanding laterally with respect to the vaginal canal 116 when the assembly 20 is positioned therein. This feature is believed to be due, at least in part, to the fact that the impermeable portion 22, by virtue of its inflexible nature, prevents the absorbent portion 24 from expanding in any direction other than in a direction which is substantially normal to the concave side face 28. Compared to a prior art absorbent device which tends to expand in every direction (including laterally) with respect to a vaginal canal within which the device is positioned - and commonly must necessarily be folded upon itself to be removed from the canal, the impermeable portion 22 of applicant's assembly 20 prohibits the absorbent portion 24 from expanding laterally within a vaginal canal and is advantageous in this respect. If desired, the absorbent portion 24 can be coated with a spermicide and left in place for a prolonged period of time (e.g. up to about six hours) following intercourse for added birth control.

The shape and inflexible nature of the impermeable portion 22 is also advantageous when positioned into place within the vaginal canal 116 from the standpoint of user comfort. In this connection and with reference again to Figs. 2 and 3, the outer edge 26 lies substantially within a plane 80 and since the impermeable portion 22 is substantially inflexible in shape, the outer edge 26 engages the tubular walls 118 of the vaginal canal 116 at points therealong which collectively conform to the oval shape of the outer edge 26 and thereby enhance user comfort. In other words and because of the inflexibility and oval shape of the outer edge 26, the tubular walls 118 are not distended laterally by any appreciable extent while maintaining sealing engagement with the tubular walls 118 along a plane.

The resultant engagement between the tubular walls 118 of the vaginal canal 116 and the planar outer edge 26 of the impermeable portion 22 is preferable over that which would result from either an inflexible impermeable portion having a circular edge or a flexible impermeable portion which assumes a collapsed or folded condition when positioned within the vaginal canal. For example, in the instance in which the outer edge of an inflexible impermeable portion is circular, the vaginal walls would necessarily distend laterally by an amount equal to the diameter of the circular outer edge and not provide as effective of a seal with the outer edge of the impermeable portion; and in the instance in which the outer edges of an impermeable portion is permitted to assume a collapsed or folded condition when positioned within the vagina canal, the resulting engagement between the tubular walls of the vaginal canal and the folded outer edge would not likely be confined to a plane.

As suggested earlier, the removal of the assembly 20 from the vaginal canal 116 is facilitated by the tension member 100 which can be pulled upon by the user. In particular and as the knot 112 is pulled upon by a user, the major portion 78 of the tension member 100 is withdrawn from the interior of the impermeable portion 22 by way of the through-openings 90, 91 so that the tension member 100 begins to lengthen, or extend, as it is pulled, or withdrawn, from the impermeable portion 22 before the impermeable portion 22 begins to move along the vaginal canal 116 toward the opening thereof. When the tension member 100 has been pulled from the interior 106 of the concave side face 106 by a sufficient distance, the looped mid-portion 99 of the tension member cord 72 engages the region 70 of the impermeable portion 22 and is prevented from exiting the interior 106 of the concave side face 28 by the region 70 so that any continued pull upon the knot 112 effects the withdrawn of the assembly 20 along the vaginal canal. Furthermore and because the looped mid-portion 99 of the tension member cord 72 is secured to the absorbent portion 24 by way of the aforedescribed stitch 102, the withdrawal of the absorbent portion 24 from the vaginal canal 116 (along with the impermeable portion 22) is assured.

It follows from the foregoing that until the assembly 20 is desired to be removed following use, the major portion 78 of the tension member 100 remains in a gathered condition within the interior 106 of the concave side face 28 of the impermeable portion 22 and between the underside of the absorbent portion 24 and the concave side face 28. The withdrawal of the tension member 100 through the through-openings 90, 91 from the Fig. 4 gathered condition to the Fig. 5 fully-extended condition is not hindered by any engagement between the tension member 100 and the surfaces of the impermeable portion 22 and the absorbent portion 24 between which (the major portion 78 of) the tension member 100 is sandwiched.

Furthermore, the impermeable portion 22 does not have to be squeezed or compressed from side-to-side in order to remove the assembly 20 from the canal 72. Further still and upon removal of the assembly 20 from the vaginal canal 72, the absorbent portion 24 can be separated from the impermeable portion 22 by pulling the components 22 and 24 apart for disposal of the absorbent portion 24 and, if desired, for recycling of the impermeable portion 22.

The tampon assembly 20 can be constructed in any of a number of sizes to accommodate a range of vaginal sizes. For example, the impermeable portion 22 can be sized so that it measures about 65 mm, 75 mm or 85 mm along its major axis 38 to accommodate vaginal canals of different sizes.

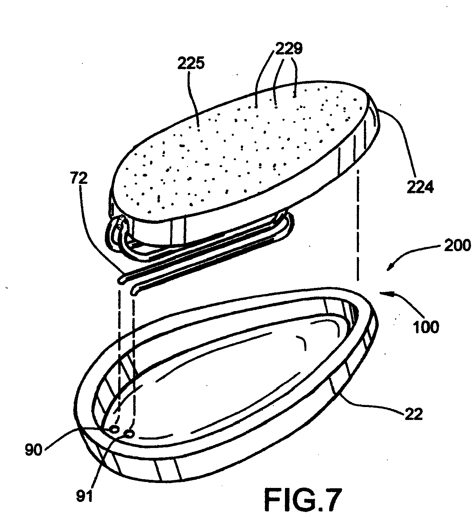

It will be understood that numerous modifications and substitutions can be had to the aforedescribed embodiment 20 without departing from the scope of the invention. For example and as mentioned earlier, an alternative form of the assembly 20 can be used to collect cells for laboratory (e.g. pap smear) purposes. With reference to Fig. 7, there is shown an alternative assembly, generally indicated 200, comprising an impermeable portion 22, a tension member 100 and an absorbent portion 224 which is securable within the interior 106 of the concave side face 28 of the impermeable portion 22. The difference between the absorbent portion 224 of the Fig. 7 assembly 200 from the absorbent portion 24 of the assembly 20 of Figs. 1-6 is that the outwardly-facing surface, indicated 225, of the absorbent portion 222 is coated with a fine abrasive substance 229 (e.g. grit) adapted to collect cells from the surface of the cervix 120 (Fig. 6) after having been worn, or used, by a user over a relatively short period (e.g. a few hours) of time. Following such a period of use, the assembly 200 is removed from the user, and because of the presence of the abrasive substance 229 across the outwardly-facing surface 225, cells from the cervix are collected upon the surface 225 for laboratory purposes.

Furthermore and although the absorbent portion 24 of the assembly 20 of Figs. 1-6 has been shown and described as including a pre-formed concave indentation 32 within its upwardly-facing surface 56, an absorbent portion of an assembly which embodies features of the present invention need not possess such an indentation 32. Accordingly, the aforedescribed embodiments 20 and 220 are intended for the purpose of illustration and not as limitation.

Further embodiments of the invention are disclosed in the following numbered clauses:
Clause 1. A tampon assembly comprising: a thin saucer-shaped impermeable portion having two opposite concave and convex side faces and having an outer edge which is oval in shape and markedly elongate in form, wherein the concave side face has an interior and the impermeable portion includes a leading end which first enters a user when positioned therein and an opposite trailing end, and the impermeable portion defines an opening therein adjacent the trailing end thereof and an inwardly-facing lip which encircles the concave side face of the impermeable portion; an absorbent portion positioned adjacent the concave side face of the impermeable portion and held therein by the inwardly-facing lip of the impermeable portion; and a tension member facilitating the withdrawal of the assembly following use, the tension member having two opposite ends and a major portion which extends between the two opposite ends, the major portion of the tension member being positioned within the interior of the concave side face of the impermeable portion, one end of the two opposite ends of the tension member protruding through the through-opening and being accessible to a user, and the other of the two opposite ends of the tension member is prevented from exiting the interior of the concave side face through the through-opening so that by pulling upon the one end of the two opposite ends of the tension member during an assembly removal process pulls the tension member to a fully-extended condition before the remainder of the assembly is withdrawn from the user.
Clause 2. The tampon assembly as defined in Clause 1 wherein the major portion of the tension member is sandwiched between the concave side face and the absorbent portion.
Clause 3. The tampon assembly as defined in Clause 1 or 2 wherein the tension member is in the form of a cord having two opposite ends and wherein the cord is folded in half upon itself so that one end of the tension member is provided by the two opposite ends of the cord and the other end of the tension member is provided by a looped mid-section of the cord.
Clause 4. The tampon assembly as defined in Clause 3 wherein the impermeable portion includes a pair of through-openings which are arranged in a side-by-side relationship with one another and which extend between the concave and convex side face of the impermeable portion, and wherein one of the two opposite ends of the cord extends through one of the pair of through-openings and the other of the two opposite ends of the cord extends through the other of the pair of through-openings so that by pulling upon the two opposite ends of the cord, the other end of the tension member is prevented from exiting the impermeable portion by way the looped mid-portion of the cord.
Clause 5. The tampon assembly as defined in Clause 4 wherein the two opposite ends of the cord which extend through the pair of through-openings are tied together in a knot external of the impermeable portion, and the knot provides a pull tab capable of being grasped for removal of the assembly.
Clause 6. The tampon assembly as defined in Clause 4 wherein the looped mid-portion of the cord is sewn to the absorbent portion at a position thereon which is located adjacent the trailing end of the impermeable portion.
Clause 7. The tampon assembly as defined in any preceding Clause wherein the absorbent pad is elongated in shape and has a trailing end which corresponds to the trailing end of the impermeable portion, and the other end of the tension member is secured to the absorbent portion at a location thereon which is disposed adjacent the trailing end of the absorbent portion.

## Claims

1. A tampon assembly comprising:
a thin saucer-shaped impermeable portion having two opposite concave and convex side faces and having an outer edge which is oval in shape and markedly elongate in form, wherein the concave side face has an interior and the impermeable portion includes a leading end which first enters a user when positioned therein and an opposite trailing end, and the impermeable portion defines an opening therein adjacent the trailing end thereof;
an absorbent portion positioned adjacent the concave side face of the impermeable portion; and
a tension member facilitating the withdrawal of the assembly following use, the tension member having two opposite ends and a major portion which extends between the two opposite ends, the major portion of the tension member being positioned within the interior of the concave side face of the impermeable portion, one end of the two opposite ends of the tension member protruding through the through-opening and being accessible to a user, and the other of the two opposite ends of the tension member is prevented from exiting the interior of the concave side face through the through-opening so that by pulling upon the one end of the two opposite ends of the tension member during an assembly removal process withdraws the tension member from the interior of the concave side face to a fully extended condition before the remainder of the assembly is withdrawn from the user.

2. The tampon assembly according to Claim 1 wherein the major portion of the tension member is sandwiched between the concave side face and the absorbent portion.

3. The tampon assembly according to any preceding claim wherein the tension member is in the form of a cord having two opposite ends and wherein the cord is folded in half upon itself so that one end of the tension member is provided by the two opposite ends of the cord and the other end of the tension member is provided by a looped mid-section of the cord.

4. The tampon assembly according to Claim 3 wherein the impermeable portion includes a pair of through-openings which are arranged in a side-by-side relationship with one another and which extend between the concave and convex side face of the impermeable portion, and wherein one of the two opposite ends of the cord extends through one of the pair of through-openings and the other of the two opposite ends of the cord extends through the other of the pair of through-openings so that by pulling upon the two opposite ends of the cord, the other end of the tension member is prevented from exiting the impermeable portion by way the looped mid-portion of the cord.

5. The tampon assembly according to Claim 4 wherein the two opposite ends of the cord which extend through the pair of through-openings are tied together in a knot external of the impermeable portion.

6. The tampon assembly according to any one of claims 4 and 5 wherein the looped mid-portion of the cord is secured to the absorbent portion.

7. The tampon assembly according to any one of claims 4 to 6 wherein the looped mid-portion is sewn to the absorbent portion at a position thereon which is located adjacent the trailing end of the impermeable portion.

8. The tampon assembly according to any one of claims 4 to 7 wherein the cord is in the form of a cotton string.

9. The tampon assembly as according to any preceding claim wherein the absorbent pad is elongated in shape and has a trailing end which corresponds to the trailing end of the impermeable portion, and the other end of the tension member is secured to the absorbent portion at a location thereon which is disposed adjacent the trailing end of the absorbent portion.

10. The tampon assembly according to Claim 9 wherein the other end of the tension member is secured to the absorbent portion with a stitch of thread.

11. A tampon assembly according to any preceding claim further comprising:
an inwardly-facing lip which encircles the concave side face of the impermeable portion;
wherein the absorbent portion positioned adjacent the concave side face of the impermeable portion is held therein by the inwardly-facing lip of the impermeable portion.

12. The tampon assembly according to any one of claims 5 to 11 wherein the knot provides a pull tab capable of being grasped for removal of the assembly.

13. A tampon assembly comprising:
a thin saucer-shaped impermeable portion having two opposite concave and convex side faces and having an outer edge which is oval in shape and markedly elongate in form; and
an absorbent portion positioned adjacent the concave side face of the impermeable portion, and the absorbent portion having an exposed surface adapted to collect cells against the surface thereof when the assembly is positioned in place.

14. The tampon assembly as defined in Claim 13 wherein the exposed surface is abrasive in nature.

15. The tampon assembly as defined in Claim 14 wherein the exposed surface includes an amount of fine grip.
